# EUROPEAN PATENT APPLICATION

(11) **EP 2 108 401 A1**
(43) Date of publication of application: **14.10.2009**
(21) Application number: 08154397.7
(22) Date of filing: 11.04.2008
(51) Int. Cl.: A61N 5/10, B29C 33/30

(54) **Organ motion compensating device and method**

(71) Applicant: ION BEAM APPLICATIONS S.A., 1348 Louvain-la-Neuve (BE)
(72) Inventor: Bentefour, El Hassane, B-3300, Tienen (BE)
(74) Representative: Van Malderen, Joëlle

(57) **Abstract**

A device for compensating the motion of a target volume of a patient to be irradiated with a radiation beam, said radiation beam being generated by a radiation source, said device (60) comprising:
• a tissue equivalent material (65) located between said radiation source and said patient;
• compensator (50) located between said radiation source and said tissue equivalent material (65), said compensator (50) capable of reproducing the distal shape of the target volume of said patient to be irradiated;
• means (61, 62, 63, 64, 66) for varying the thickness of said tissue equivalent material (65) in order to compensate the motion of said target volume.

## Description

### Technical Field

The present invention relates to the field of Radiotherapy, and in particular to the field of Hadron therapy, i.e. radiation therapy using strongly interacting particles, such as protons or carbon ions for example. More particularly, the present invention relates to an apparatus and method for obtaining a patient specific distal contour to be used as a compensator. Furthermore, the present invention relates to a device for compensating the movement of a target volume of a patient to be irradiated with a charged particle beam. This patient specific distal contour is capable of reproducing the distal shape of said target volume and of modifying the distribution of absorbed dose over the radiation field of a radiation beam.

### Description of Related Art

Treatment of a tumour in a patient is typically performed in conventional radiotherapy, as well as in hadron therapy, by planning the radiation angles and dosage of the radiation beam to be delivered to the patient. However, the planning of such angles and dosage takes into account particular safety factors with respect to the patient's healthy organs which are traversed by the beam during the treatment. As a consequence, volume definition of the tumour of the patient is one of the most important aspects for 3D treatment planning, as well as for accurate dose delivering.

It is well established that hadrons (i.e. neutrons, protons, pions, ions such as carbon ions) have certain physical advantages with respect to X-rays or gamma rays in the radiotherapy field. It is well known, for example, that protons of a given energy, i.e. forming a monoenergetic proton beam, have a certain range and do not penetrate beyond that range. Furthermore, they deposit their maximum value of radiation energy in the so-called Bragg Peak, which corresponds to the point of greatest penetration of the radiation in a target volume. Since the Bragg peak position depends on the energy of the hadron beam, it is evident that by precisely controlling and modifying the energy one can place the Bragg Peak at a suited depth of a tumour so as to administer the greatest radiation energy to that point and spare, by contrast, healthy tissue surrounding said tumour. Moreover, by combining several monoenergetic proton beams of different energies (i.e. performing the range modulation) it is possible to extend the Bragg Peak in order to match the thickness of the tumour and irradiate the target with a uniform dose while controlling the dose load on surrounding healthy tissue and critical organs.

Typically, a hadron therapy system mainly comprises an accelerator that accelerates hadrons, e.g. protons or carbon ions, in the form of a beam to a desired energy level and, through a beam transport system, delivers the hadron beam to a target volume, i.e. a tumour. The last structure along the beam transport system and before said target comprises a nozzle which drives and shapes the hadron beam toward a specific target isocenter.

However, whether on the one hand the Hadron therapy has advantages over conventional radiotherapy, on the other hand it requires special equipment in order to combine together hadrons of different energies. Furthermore, special equipment is also required to shape the hadron beam so as to match, as much as possible, the shape, size and location of the tumour. In particular, irregular shape field boundaries are typically obtained by providing shadow blocks on a shadow tray between the patient and the nozzle. Furthermore, irregular intensity across the cross-section of the target is obtained by means of wedge filters or compensating filters which are located between the patient and the nozzle.

Compensating filters, or compensators, are usually located in a shielding slot between the nozzle of a radiation apparatus and the patient. Compensators may be made of any material with a certain thickness, even if lead made compensators are much more practical and compact. Such a compensator is shaped in such a way as to match the tumour's anatomical shape and has a certain thickness in order to attenuate the beam by a precise amount. A compensator suitable for conventional radiotherapy typically modifies the distribution of absorbed dose over the radiation field in such a way as to ensure a uniform target volume regardless of the shape of this target. A compensator suitable for Hadron therapy (typically referred as a range compensator) is computed and shaped by the TPS, in order to fit the distal range of the spread out Bragg peak to the distal shape of the tumour.

However, the manufacture process of traditional compensators is, according to prior art, time-consuming and very expensive, since each single compensator is exclusively manufactured for a single tumour at single treatment angle.

It is known from document US 4,623,288 an apparatus and method for fabricating a compensator. This device comprises: a stylus for tracing a patient's contour and a cutter for cutting a form. The stylus and the cutter are coupled in such a way that movements of the stylus generate movements of the cutter, the latter defining a cavity in the form corresponding to the traced portion of the patient. A molding process creates the compensator by filling said cavity with a suitable material. However, this device it is very imprecise since it is related to human operation and uncomfortable for the patient. Moreover, the described process is time consuming due to fill-in step and the subsequent dry step.

It is also known from document US 5,014,290 a system for generating radiation blockers by means of cutter means. This system further comprises a computer for generating cutter control signals, according to digitized processed images received by said computer from a fluoroscopic simulator. However, the manufacture process for obtaining radiation blocks, according to this document, is time-consuming and expensive. Furthermore, this device and process may lack of precision during the step of turning soft foam into robust material.

However, both cited documents can only compensate for irregular patient body surfaces but not the distal shape of a tumour.

Another well known problem in radiation therapy, relates to the organ motion of a patient which makes even more difficult the use of known compensators. In fact, when the radiation treatment is applied to tumours that are located, for example, in the abdominal or thoracic regions, the delivery of such treatment is constantly affected by tumours displacements caused by normal respiration of the patient. As a consequence, when the target volume moves from its initial position, the thickness of such a compensator is no more the required one, whereby a wrong delivery of the radiation treatment is caused. Since traditional compensators have fixed thickness, current solutions for compensating tumour displacements due to organ motions are very complex and have several disadvantages.

Several solutions are currently implemented into the clinical practice for compensating such target volume displacements. For example, advanced methods for managing respiratory displacement of a tumour during treatment include voluntary breath-hold and forced breath-hold methods. However, breath-hold methods have the drawback of being very uncomfortable for patients and unsafe since the patient could not respect the breath holding instructions.

Another current method is the beam gating method (see for example documents US 5727554 and EP1793738**),** wherein the beam is switched off when the organ of the treated patient moves away from the target position and turned on when the target regains wanted position. Even if this solution optimizes the sparing of healthy tissues, it does increase the treatment time with a factor of three and will not compensate for movements of the patient unrelated to respiration. Furthermore, these techniques may be uncomfortable for patients, in particular for patients having pulmonary problems. Another current solution consists in real-time tracking technique, wherein the range of the beam is adjusted by changing the beam energy depending on the organ motion by means of a multi-leaf collimator. This last solution is however quite complex and very expensive as well.

The present invention aims to provide an apparatus and method for obtaining a compensator which is time efficient, reliable, precise and comfortable for patients.

The present invention also aims to provide a compensator which overcomes the drawbacks of prior art.

More particularly, the present invention aims to provide a compensator that makes easier and faster to compensate for tumor displacements due to organ motions.

### Summary of the Invention

The present invention overcomes the shortcomings of the conventional art and may achieve other advantages not contemplated by conventional devices.

According to a first aspect of the invention, there is provided an apparatus for obtaining a compensator to be used for compensating the motion of a target volume of a patient to be irradiated with a radiation beam, said patient specific distal contour reproducing the shape of said target volume and capable of modifying the distribution of absorbed dose over the radiation field of a radiation beam, this radiation beam being generated by a radiation apparatus, said radiation apparatus being capable of processing and providing data of the tri-dimensional geometry of the target volume to be irradiated, the apparatus characterized in that it comprises:
- a first mechanism, for obtaining a tri-dimensional target distal contour mold, comprising:
   ■ a phantom made of a plurality of parallel rods, said rods capable of being individually moved in a parallel direction to the direction of said radiation beam;
   ■ driving means for independently moving said rods according to said data in order to obtain the tri-dimensional target distal contour mold;
- a second mechanism for printing said tri-dimensional target distal contour mold over a suitable substrate for obtaining said compensator.

Preferably, said tri-dimensional target distal contour mold comprises means capable of bringing and keeping said mold at a specific required temperature close to the melting point of said substrate.

Advantageously, said substrate is a polycarbonate plastic powder.

Optionally, said parallel rods are cylindrically shaped rods.

Advantageously, said rods are made of magnetized metal and are magnetized enough so that when they are in contact to each other, they can easily adhere to each other.

Optionally, said parallel rods are brass rods, each having on both faces rail-like grooves, whereby it is possible to slide each rod in each other.

Preferably, the apparatus further comprises a member capable of wrapping and keeping in a fixed position said plurality of parallel rods.

Advantageously, said member can be set in a lock and in an unlock position by means of a lock, whereby, when said member is locked, said plurality of parallel rods is fixed, while when it is unlocked said plurality of parallel rods remains in its initial positions in such a way that each rod may move vertically when a sufficient vertical force is exerted on it by said driving means.

More advantageously, said member is comprised of a thin unstretchable metallic sheet.

Preferably, said metallic sheet comprises a thin wire which is electrically conductive and insulated with a temperature resistant varnish, whereby it is possible to control the temperature of said mold, by applying an electrical current to said thin wire.

According to a second aspect of the present invention, there is provided a method for obtaining a compensator to be used for compensating the motion of a target volume of a patient to be irradiated with a radiation beam, said compensator reproducing the shape of said target volume and capable of modifying the distribution of absorbed dose over the radiation field of a radiation beam, this radiation beam being generated by a radiation source, the method characterized in that it comprises the steps of:
- providing a phantom made of a plurality of parallel rods, said rods capable of being individually moved in a parallel direction to the direction of said radiation beam;
- providing driving means for independently moving each of said rods;
- acquiring data of the tri-dimensional geometry of the target volume to be irradiated;
- providing said driving means with said data in the form of digital signals;
- running said driving means based on said digital signals by vertically moving one or more of said parallel rods in order to conform said tri-dimensional geometry of a target volume to be irradiated and forming a tri-dimensional target distal contour mold;
- bringing said tri-dimensional target distal contour mold to a specific temperature;
- providing means for printing said tri-dimensional target distal contour mold over a suitable substrate for obtaining said compensator.

Preferably, said method further comprises the step of cool down said patient specific distal contour mold after the printing step.

According to a third aspect of the present invention there is provided a device for compensating the motion of a target volume of a patient to be irradiated with a radiation beam, said radiation beam being generated by a radiation source, said device comprising:
- a tissue equivalent liquid material located between said radiation source and said patient;
- a compensator located between said radiation source and said tissue equivalent material, said compensator capable of reproducing the distal shape of the target volume of said patient to be irradiated;
- means for varying the thickness of said tissue equivalent material in order to compensate the motion of said target volume.

Preferably, said means for varying the thickness of said tissue equivalent material comprises a piston capable of moving said compensator in equal amplitude but opposite direction with respect to the organ motion.

More preferably, said means for varying the thickness of said tissue equivalent material also comprises elastic rings capable of extending or reducing the volume of said reservoir tank, accordingly to movements of said piston.

Advantageously, said tissue equivalent material is water.

### Brief Description of the Drawings

**Fig. 1** is a schematic view of an apparatus for obtaining a patient specific distal contour, according with a first aspect of the present invention.

**Fig. 2** is a diagram block representing the steps of a method for obtaining a patient specific distal contour, according to a second aspect of the present invention.

**Fig. 3** represents a perspective view of a target distal contour mold.

**Fig. 3a** and **Fig. ]3b** represent two different variants of the target distal contour mold of **Fig.3****.**

**Fig. 4** represents a perspective view of an exemplary tri-dimensional patient specific distal contour.

**Fig. 5** represents a device for compensating the motion of a target volume, according to a third aspect of the present invention.

### Detailed Description of the Invention

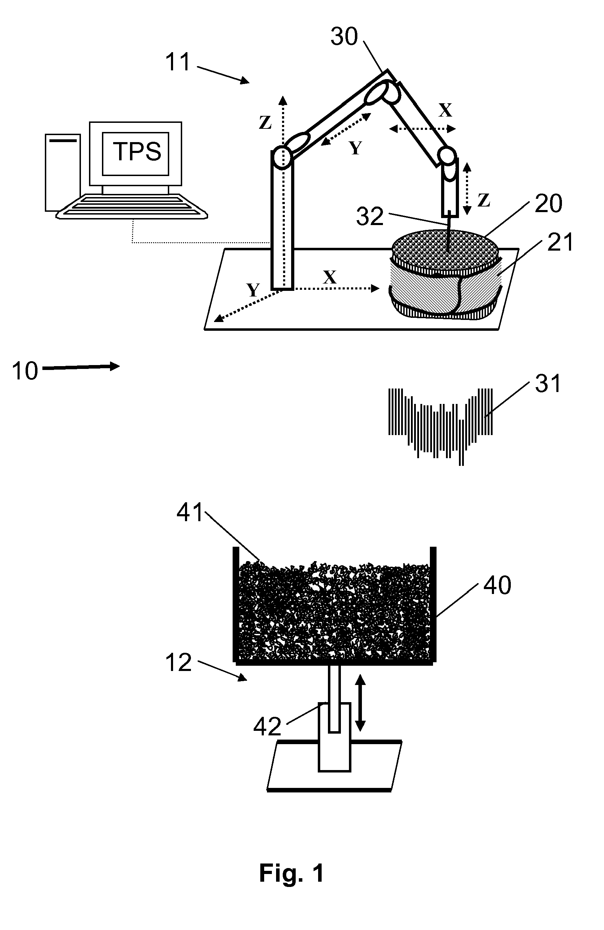

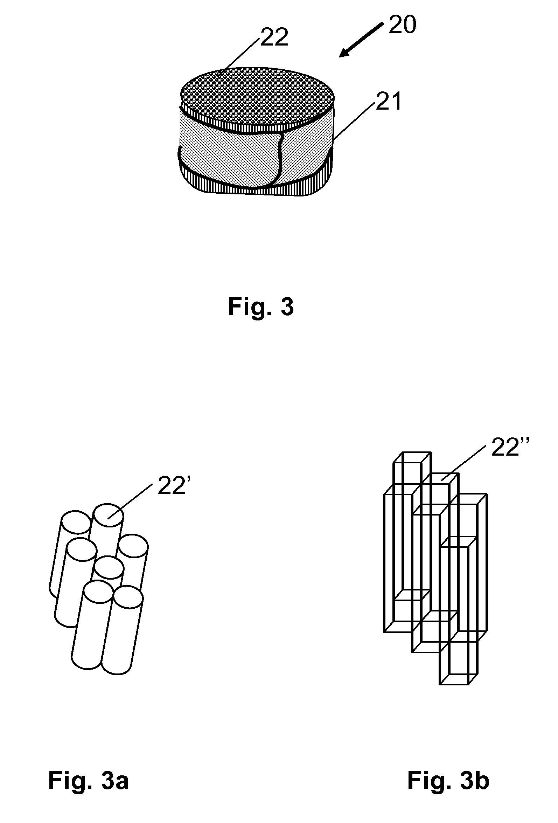

**Fig. 1** shows an apparatus for obtaining a patient specific distal contour according to a first aspect of the invention. The apparatus 10 comprises a first mechanism 11 for obtaining a target distal contour mold, referred hereinafter as mold. Said first mechanism 11 comprises:
- a phantom 20 made of a plurality of parallel metallic rods 22, as clearly shown in **Fig.3****.** Said rods 22 are capable of being individually moved along a direction parallel to the direction of a radiation beam emitted by a radiation apparatus;
- driving means 30, such as a robotic arm, for vertically displacing each individual rod 22 of said phantom 20, according to data provided by a treatment planning system TPS, in order to shape said phantom 20 corresponding to a tri-dimensional distal contour of the target to be irradiated.

According to a first embodiment of the first aspect present invention, as shown in **Fig.3a****,** these metallic rods 22' are cylindrically shaped with a diameter of 2 mm and length of 30 cm. Rods 22' are made of slightly magnetized metal and are magnetized enough so that when they are in contact to each other, they can easily adhere (stick) to each other. Their adhesion is strong enough to resist to the gravitation field so that it is possible to build-up, using several rods, a solid cylinder with a diameter of 25 cm and 20 cm length.

According to a second embodiment of the first aspect of the present invention, as shown in **Fig.3b****,** rods 22" are made of brass with a small square section (or hexagonal section) of about 6.25 mm2 and length of 20 cm. Both faces of each rod 22" are machined with rail-like grooves. Said rail-like grooves, machined in the brass rods 22", serves for sliding the rods 22" in each other to build a sort of net-like cubby-structure. When rods 22" are assembled together, they form a filled cube of 25 cm x 25 cm x 20 cm.

By referring again to Fig.1, said robotic arm 30 ends up with a robust stainless steel needle 32 of 1 mm thickness and is controlled by processing means, such as a computer. Said robotic arm 30 is capable of moving the stainless steal needle 32 in a horizontal plane of 25 cm per 25 cm, with step of 1 mm and along a vertical axis up to 15 cm with step of 0.5 mm. The phantom 20 further comprises a member 21, such as a belt, capable of wrapping and keeping in a fixed position this plurality of rods 22. Said member 21 can be set in a lock and in an unlock position, so as when it is locked, rods 22 are fixed, while when it is unlocked, each rod 22 remains in its initial positions but may move vertically when a strong enough vertical force is exerted on it. Said member 21 comprises a thin unstretchable metallic sheet with a thickness of around 3 mm and width of 10 cm. This metallic sheet comprises a thin wire having a thickness of about 500 µm and is electrically conductive and insulated with a temperature resistant varnish.

When the member 21 is unlocked, the robotic arm 30 can exert by means of the needle 32 along the z-axis of these rods 22 a force greater than the friction between a single rod and the surrounding rods of the phantom 20. So as when the tridimensional distal shape of the target volume has been reproduced, the member 21 is locked and finally a rigid multi-rods mold is obtained. In this manner, it is possible to mimic about the entire range of the dimensions of well known traditional compensators in Hadron therapy. It should be noted that a typical dimension of such a compensator is about 15 cm x 15 cm x 10 cm and is typically manufactured by using a milling machine, which is a time consuming and expensive process.

By applying an electrical current to the thin wire of said member 21, it is possible to control, by means of control means (not shown), the temperature of said mold 31 which may be varied between 30°C and 160°C. The total electrical resistance of this thin wire does not exceed 100 Ohm. Furthermore, by means of said control means, it is possible to drive and stabilize its temperature up to the typical melting temperature of plastics (~ 160 °C) with a precision of 0.1 °C. Moreover, the lower part of the rods 22 (or eventually the complete rods surfaces) are painted or coated with a material that resists in the earlier temperature range and disfavors the adhesion of plastics.

After the mold 31 has been obtained, its temperature is increased to around the melting point of a polycarbonate based plastics (~ 155°C).

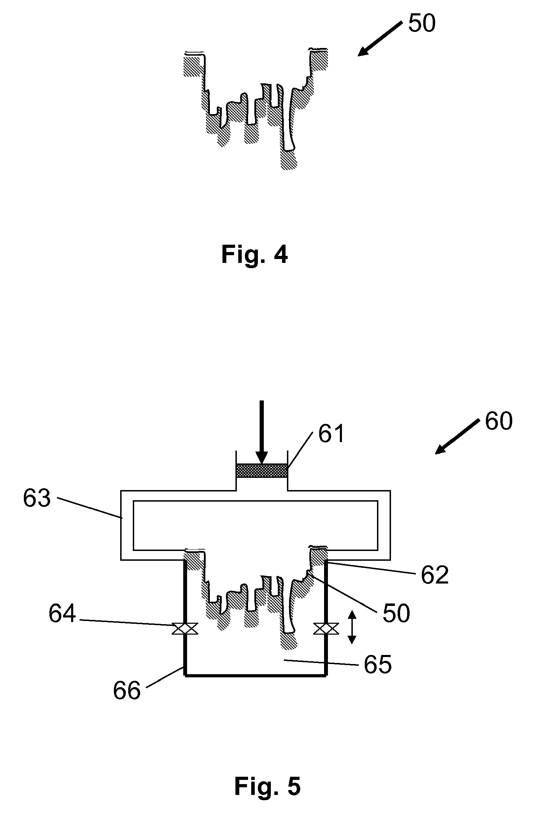

By referring again to **Fig.1****,** the apparatus 10 further comprises a second mechanism 12 for printing said mold 31 over a suitable substrate 41, as described hereinafter. Said substrate 41 is, according to a preferred embodiment, polycarbonate plastic powder 41 which is contained in a movable reservoir 40. Said reservoir 40 is driven by an automated arm 42 and pushed progressively against said multi-rods mold 31. The contact between said multi-rods mold 31 and the powder 41 lasts until the powder 41 melts and takes the exact form of the multi-rods mold 31, without adhering, with a thickness of about 5 mm to 10 mm (depending on the application). In order to speed up the process a second container (not shown) filled with cold water may be provided for replacing the reservoir 40. In this manner, it is possible to cool down the multi-rods mold 31 and to therefore harden the polycarbonate plastic powder 41 for easily coming out from the mold 31. Finally, when the polycarbonate plastic powder 41 is hardened and removed from said mold 31, the patient specific distal contour 50 is obtained, as shown in **Fig.4****.**

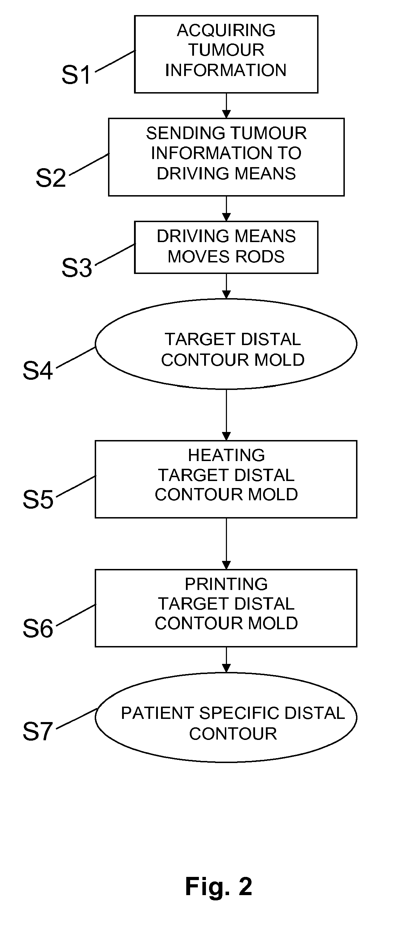

**Fig. 2** is a diagram block representing the steps of a method for obtaining a patient specific distal contour, according to a second aspect of the present invention. When the apparatus 10 is in use, the treatment planning system acquires (step S1) data of the tridimensional geometry of the target volume and provides (step S2) said robotic arm 30 with digital signals corresponding to said data. The robotic arm 30 executes (step S3) said digital signals and accordingly by means of needle 32 moves rods 22 of the phantom 20 so as to obtain ((step S4) the mold 31, the latter reproducing a given patient specific distal contour according to acquired data of the tridimensional geometry of the target volume. Said robotic arm 30 executes the computer commands in order to optimize the time necessary to obtain this mold 31. Subsequently, by means of said control means it is possible to drive and stabilize the temperature (step S5) of said mold 31 up to the typical melting temperature of plastics (~ 160 °C) in order to print (step S6) said mold 31 over a polycarbonate plastic powder 41. The contact between said mold 31 and the powder 41 lasts until the powder 41 melts and takes the exact form of the mold 31, without adhering, with a thickness of about 5 mm to 10 mm (depending on the application). In order to speed up the process a second container (not shown) filled with cold water may be provided for replacing the reservoir 40. In this manner, it possible to cool down the mold 31 and therefore harden the polycarbonate plastic powder 41. Finally, the hardened polycarbonate plastic powder 41 is removed from the mold 31 and the patient specific distal contour 50 is obtained (step S7).

It should be noticed that the manufacture process of such a patient specific distal contour is cost-effective and precise, as well as much easier, cheaper and faster with respect to prior art manufacture processes. Furthermore, such a patient specific distal contour is furthermore ecologic since is may be easily recycled.

It should be noticed that this patient specific distal contour 50 may be used as a static compensator (together with a tissue equivalent material located between said patient specific distal contour 50 and the patient) or it may be used for having a dynamic compensator for compensating for tumor displacements as hereinafter described.

**Fig.5** shows a device for compensating the target displacement due to organ motions during a treatment, according to a third aspect of the present invention. In order to be able to compensate for organ motions, it is necessary to have firstly an imaging system that reads the organs motion online during treatment and translates it into electrical signals. Such an imagining system may be for example:
- an echograph that uses ultrasound waves to detect organs motion and allows the quantification of this motion;
- a spirometer that measures the quantity of air unhealed or up-healed by a patient and performs, via patient specific calibration curves, a conversation into organs motion;
- a fluoroscope that uses x-ray imaging in order to monitoring the organs motion.

The device 60 is adapted to cooperate with a radiation therapy apparatus, and more precisely with a passive proton therapy beam delivery apparatus capable of acquiring information about tumour displacements. The device 60 comprises:
- a closed hermetic container 62 filled with a tissue equivalent liquid material 65;
- a patient specific distal contour 50 (as previously described);
- means 61, 64 for varying the level of said tissue equivalent liquid material 65 contained in said container 62.

Said tissue equivalent liquid material 65 is, according to a preferred embodiment, water. Said container 62 is located between the nozzle of said radiation therapy apparatus and the target volume to be irradiated, and comprises a reservoir tank 66 and a pipe 63 wherein water can freely circulate. The patient specific distal contour 50 is located between said exit of said radiation therapy apparatus and said reservoir tank 66 and is capable of reproducing the distal shape of the target volume of said patient to be irradiated. The patient specific distal contour 50 is contained into said reservoir tank 66 by means of support means (not shown), so as it can be easily inserted and removed, and when it is inserted, it forms, together with said pipe 63 and said reservoir tank 66, the container 62.

Said means 61, 64 for varying the level of water is arranged so as to adjust the level (i.e. the water equivalent thickness) of the water contained in said reservoir tank 66 and to compensate the motion of said target volume. Acquired information about tumour displacements is transmitted, on the form of electrical signals, to a driving motor (not shown) that applies a mechanical force to a piston 61, the latter hermetically closing the pipe 63. Said piston 61 translates said mechanical force into the opposite of the organ motion, and consequently moves the patient specific distal contour 50 in equal amplitude but opposite direction with respect to tumour displacements. Said means for varying the level of water 61, 64 also comprises elastic rings 64 capable of extending or reducing the volume of said reservoir tank 66, accordingly to translations of said piston 61. As a consequent, the level of water circulating in said container 62 may be varied according to movement of piston 61 and said elastic rings 64, so that when the organ moves away from the target position, the level (i.e. the water equivalent thickness) of water contained into the reservoir tank 66 can be adjusted allowing for higher or smaller range during the delivery. As result, the organ motion will not affect the quality of the treatment using the passive proton therapy beam delivery methods.

One or more embodiments of the present invention have been described in detail with reference to the attached figures. It is evident that the invention is only limited by the claims, since the figures described are only schematic and therefore non-limiting. In the figures, the size of some of the elements may be exaggerated and not drawn on scale for illustrative purposes. The dimensions and the relative dimensions do not necessarily correspond to actual reductions to practice of the invention. Further, those skilled in the art can recognize numerous variations and modifications of this invention that are encompassed by its scope. Accordingly, the description of preferred embodiments should not be deemed to limit the scope of the present invention.

It is evident for those skilled in the art that with the necessary adaptations other embodiments of the invention may be easily conceived even for other applications.

Furthermore, the terms first, second and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims may be used for descriptive purposes and not necessarily for describing relative positions. The terms so used are interchangeable under appropriate circumstances and the embodiments of the invention described herein can operate in other orientations than described or illustrated herein. For example "underneath" and "above" an element indicates being located at opposite sides of this element.

It is to be noticed that the term "comprising", used in the claims, should not be interpreted as being restricted to the means listed thereinafter; it does not exclude other elements or steps. Thus, the scope of the expression "a device comprising means A and B" should not be limited to devices consisting only of components A and B. It means that with respect to the present invention, the only relevant components of the device are A and B.

## Claims

1. An apparatus (10) for obtaining a compensator (50) corresponding to the distal shape of a target volume of a patient to be irradiated with a radiation beam, said compensator (50) to be used for compensating the motion of a target volume and capable of modifying the distribution of absorbed dose over the radiation field of said radiation beam, this radiation beam being generated by a radiation apparatus, said radiation apparatus being capable of processing and providing data of the tri-dimensional geometry of said target volume, the apparatus (10) **characterized in that** it comprises:
• a first mechanism (11), for obtaining a tri-dimensional target distal contour mold (31), comprising:
■ a phantom (20) made of a plurality of parallel rods (22, 22', 22"), said rods (22, 22', 22") capable of being individually moved in a parallel direction to the direction of said radiation beam;
■ driving means (30) for independently moving said rods (22, 22', 22") according to said data in order to obtain the tri-dimensional target distal contour mold (31);
• a second mechanism (12) for printing said tri-dimensional target distal contour mold (31) over a substrate (41) for obtaining said compensator (50).

2. The apparatus (10) according to claim 1, **characterized in that** said tri-dimensional target distal contour mold (31) comprises means capable of bringing and keeping said mold (31) at a specific required temperature close to the melting point of said substrate (41).

3. The apparatus according to claim 1 or 2, **characterized in that** said substrate (41) is a polycarbonate plastic powder.

4. The apparatus according to any of the preceding claims, **characterized in that** said parallel rods (22, 22', 22") are cylindrically shaped rods (22').

5. The apparatus according to claim 4, **characterized in that** said rods (22, 22', 22") are made of magnetized metal and are magnetized enough so that when they are in contact to each other, they can easily adhere to each other.

6. The apparatus according to claim 1, **characterized in that** said parallel rods (22, 22', 22") are brass rods (22"), each having on both faces rail-like grooves, whereby it is possible to slide each rod in each other.

7. The apparatus according to any of the previous claims, **characterized in that** it comprises means for keeping in a fixed position said plurality of parallel rods (22, 22', 22").

8. The apparatus according to claim 7, **characterized in that** said means is a member (21) capable of wrapping.

9. The apparatus according to claim 8, **characterized in that** said member (21) can be set in a lock and in an unlock position by means of a lock, whereby, when said member (21) is locked, said plurality of parallel rods (22, 22', 22") is fixed, while when it is unlocked said plurality of parallel rods (22, 22', 22") remains in its initial positions in such a way that each rod may move vertically when a sufficient vertical force is exerted on it by said driving means (30, 32).

10. The apparatus according to claim 8 or 9, **characterized in that** said member (21) is comprised of a thin unstretchable metallic sheet.

11. The apparatus according to claim 10, **characterized in that** said metallic sheet comprises a thin wire which is electrically conductive and insulated with a temperature resistant varnish, whereby it is possible to control the temperature of said mold (31), by applying an electrical current to said thin wire.

12. A method for obtaining a compensator (50) corresponding to the distal shape of a target volume of a patient to be irradiated with a radiation beam and to be used for compensating the motion of said target volume, said compensator (50) capable of modifying the distribution of absorbed dose over the radiation field of said radiation beam, this radiation beam being generated by a radiation source, the method **characterized in that** it comprises the steps of:
• providing a phantom (20) made of a plurality of parallel rods (22, 22', 22"), said rods (22, 22', 22") capable of being individually moved in a parallel direction to the direction of said radiation beam;
• providing driving means (30, 32) for independently moving each of said rods (22, 22', 22") ;
• acquiring data (S1) of the tri-dimensional geometry of the target volume to be irradiated;
• providing (S2) said driving means (330, 32) with said data in the form of digital signals;
• running (S3) said driving means (30, 32) based on said digital signals by vertically moving one or more of said parallel rods (22, 22', 22") in order to conform said tri-dimensional geometry of a target volume to be irradiated and forming (S4) a tri-dimensional target distal contour mold (31);
• bringing (S6) said tri-dimensional target distal contour mold (31) to a specific temperature;
• providing means (12, 40, 42) for printing (S6) said tri-dimensional target distal contour mold (31) over a suitable substrate (41) for obtaining (S7) said compensator (50).

13. The method of claim 12, **characterized in that** it further comprises the step of cool down said patient specific distal contour mold (31) after the printing step (S6).

14. A device for compensating the motion of a target volume of a patient to be irradiated with a radiation beam, said radiation beam being generated by a radiation source, said device (60) comprising:
• a tissue equivalent liquid material (65) located between said radiation source and said patient;
• a compensator (50) located between said radiation source and said tissue equivalent material (65), said compensator (50) capable of reproducing the distal shape of the target volume of said patient to be irradiated;
• means (61, 62, 63, 64, 66) for varying the thickness of said tissue equivalent material (65) in order to compensate the motion of said target volume.

15. The device according to claim 14, **characterized in that** said
means (61, 62, 63, 64, 66) for varying the thickness of said tissue equivalent material (65) comprises a piston (61) capable of moving said compensator in equal amplitude but opposite direction with respect to the organ motion.

16. The device according to claim 14 or 15, **characterized**
**in that** said means (61, 62, 63, 64, 66) for varying the thickness of said tissue equivalent material (65) also comprises elastic rings (64) capable of extending or reducing the volume of said reservoir tank (66), accordingly to movements of said piston (61).

17. The device according to any of the claims 14 to 16, **characterized in that** said tissue equivalent material (65) is water.
